# EUROPEAN PATENT SPECIFICATION

(11) **EP 0 447 464 B1**
(45) Date of publication and mention of the grant of the patent: **05.08.1998**
(21) Application number: 90900722.1
(22) Date of filing: 08.12.1989
(51) Int. Cl.: C12Q 1/68, G01N 33/543, G01N 33/58, G01N 33/571

(54) **AMPLIFIED DNA ASSAY**
AMPLIFIZIERTER DNS-ASSAY
EXAMEN PAR ADN AMPLIFIES

(30) Priority: 09.12.1988 AU 18/89; 04.07.1989 AU 5080/89
(43) Date of publication of application: 25.09.1991
(73) Proprietor: AMRAD CORPORATION LIMITED, Kew, VIC 3101 (AU)
(72) Inventor: KEMP, David, James, North Balwyn, VIC 3104 (AU); FOOTE, Simon, James, Flemington, VIC 3031 (AU); PETERSON, Michael, Gregory, Lower Templestowe, VIC 3107 (AU); SAMARAS, Nicholas, Caulfield, VIC 3162 (AU); SMITH, Donald, Berwickshire TD13 5XE (GB)
(74) Representative: Woods, Geoffrey Corlett
(86) International application number: AU8900526
(87) International publication number: WO9006374

(56) References cited:
- EP-A- 0 223 618
- EP-A- 0 224 126
- EP-A- 0 297 379
- EP-A- 0 329 822
- WO-A-89/03891
- AU-A- 1 193 788
- AU-A- 2 735 988
- AU-A- 3 562 289
- TIBS, vol. 14, April 1989, Elsevier Science Publishers Ltd. (GB); K. STRUHL, pp. 137-140/
- PROCEEDINGS OF THE NATL. ACADEMY OF SCIENCES USA, vol. 81, October 1984, Washington, DC (US); A.G. HINNEBUSCH, pp. 6442-6446/
- PROCEEDINGS OF THE NATL. ACADEMY OF SCIENCES USA, vol. 86, April 1989, Washington, DC (US); D.J. KEMP et al., pp. 2423-2427/
- NUCLEIC ACIDS RESEARCH, vol. 17, no. 14, 1989; A.M. LEW et al., pp. 5859-5860/
- NUCLEIC ACIDS RESEARCH, vol. 16, no. 7, 11 April 1988, IRL Press, Oxford (GB); S. STAHL et al., pp. 3025-3038/
- NUCLEIC ACIDS RESEARCH, vol. 16, no. 23, 1988, IRL Press, Oxford (GB); A.
- SYVANEN et al., pp. 11327-11338/
- CHEMICAL ABSTRACTS, vol. 109, no. 19, 07 November 1988, Columbus, OH (US); A. YAMANE et al., p. 219, no. 164992v/
- Nucleic Acids Research, Symposium Series No. 20, 1988, p.91-92

## Description

The present invention relates generally to the capture and detection of amplified target DNA in a sample. More particularly, the present invention relates to a single or multi-step amplified DNA assay (ADA) and the use thereof in the rapid capture and detection of target DNA in a sample, such as in the detection of a pathogen.

The polymerase chain reaction (PCR) system (1,2) for amplifying specified segments of DNA has already proved to be of great value in experimental biology (1-8, see also Australian Patent Applications Nos. 55322/86, 55323/86, 69962/87 and 77298/87 in the name of Cetus Corporation). In the PCR procedure, a sample containing the DNA of interest is repetitively cycled through three temperatures. This results successively in denaturation of the DNA, annealing of synthetic oligonucleotides at the boundaries of the sequence of interest and the extension of the oligonucleotides by the DNA polymerase from Thermus aquaticus (Taq) (2). The exponentially amplified DNA segment can then be detected by simple procedures such as staining with ethidium bromide after agarose gel electrophoresis, or by hybridisation or sequencing to ensure that it is the expected sequence (1-6).

The PCR system should rapidly replace conventional procedures in many areas of mass screening (7). One of these is the detection of pathogens because of the generality of the technique and its exquisite sensitivity. Testing blood smaples for human immunodeficiency virus (HIV) sequences is one such area in which preliminary studies have been reported (8). Other areas include epidemiology and human genetic applications such as HLA typing and screening for genetic diseases. However, current procedures for detection of the products of PCR reactions are not well suited to mass screening as they generally require gel electrophoresis. Further, artefactual DNA molecules resulting from such events as dimerization of the primers or misincorporation of primers into irrelevant sequences can readily arise and so hybridisation or sequence information is necessary to identify a molecule with certainty. Hence, an assay system for detecting DNA amplified by the PCR procedure that is highly specific, rapid, readily, applicable to mass screening, suitable for any known sequence and uses equipment already available in many laboratories would be advantageous.

Syvänen et al (1988) Nucleic Acids Research 16 11327-11338 discloses the use of oligonucleotides modified with biotin in the 5'-end as primers in polymerase chain reaction (PCR)-amplification. This results in the synthesis of 5'-biotinylated DNA molecules, which are detected by hybridization to a labelled probe in solution. The formed hybrids are collected on an avidin-matrix by mediation of the biotin residue of the target molecules. The affinity-based hybrid collection method is quantitative and makes it possible to measure the amount of DNA produced in the PCR-amplification.

GB-A-2 202 328 discloses a rapid and sensitive method for assaying nucleic acids by hybridization in which the detector probes are modified primers incorporated into copies of the target nucleic acid before hybridization reaction, as well as a reagent combination and a kit therefor. There is also provided a method for assaying nucleic acids by hybridization in which capturing probes are modified primers incorporated into copies of the target nucleic acids before hybridization.

Yamane et al (1988) Nucleic Acids Symp. Ser. 1988 20 91-92 discloses a rapid and highly sensitive method for the detection of DNA. A specific sequence is amplified by the polymerase chain reaction with 2 different primers. One of the primers is modified with an affinity label (e.g. biotin) at the 5' end through the hydroxyl group. The other primer is modified for detection using radioisotopes or fluorphores at the 5' end. This method was used to detect human β-globin gene segments.

The present invention provides a method for capturing and detecting target DNA, which method comprises subjecting said target DNA to a polymerase chain reaction using a set of oligonucleotide primers selected to be complementary to the strands of said target DNA and wherein one of the primers of said set of oligonucleotides bears a first ligand containing a site for a double-stranded DNA-specific binding protein and the other of the primers bears a second ligand or a label, contacting the amplified double-stranded DNA with a solid substrate having the double-stranded DNA-specific DNA binding protein for said first ligand immobilized thereon and detecting the second ligand or label while the amplified double-stranded DNA is bound to said solid substrate to indicate the presence of the amplified double-stranded DNA.

In one embodiment, the present method first optionally amplifies target DNA by the polymerase chain reaction procedure using a first set of oligonucleotide primers selected to be complementary to the strands of said target DNA. The first PCR is optional to the extent that there may be an amount of target DNA sufficient to enable the practitioner to proceed to the next step without a first amplification. The target DNA, amplified or not, is amplified by the polymerase chain reaction procedure using a second set of oligonucleotide primers, the primers of said second set being selected to be complementary to the strands of said target DNA and being nested between the primers of said first set, and wherein one of the primers of said second set bears a first ligand containing a site for a double-stranded DNA-specific DNA-binding protein and the other of the primers of said second set bears a second ligand or label. The amplified DNA is contacted with a solid substrate having a double-stranded DNA-specific binding protein for said first ligand immobilized thereon. Thus, the invention includes a method for capturing and detecting target DNA in a sample, which method comprises amplifying said target DNA, if present in said sample, by a polymerase chain reaction procedure using a first set of oligonucleotide primers selected to be complementary to the strands of said target DNA, and detecting the amplified target DNA by a method according to the preceding paragraph wherein the set of primers employed in said method according to the preceding paragraph is nested between said first set of oligonucleotide primers. The first and second amplification steps can occur in either a single reaction mixture or two successive reaction mixtures.

Another aspect of the present invention is directed to a test kit for capturing and detecting target DNA in a sample by the amplified DNA assay, said kit comprising in compartmental form, a first container containing a set of oligonucleotide primers selected to be complementary to the strands of said target DNA and wherein one of the primers of said set of oligonucleotides bears a first ligand containing a site for a double-stranded DNA-specific binding protein, and the other of the said primers bears a second ligand or a label; and,
in said first container or in a second container, reagents for a polymerase chain reaction, a solid substrate coated with the double-stranded DNA-specific binding protein, and a means for detecting amplified double-stranded DNA bound to said solid substrate.

The target DNA may be subjected directly to one or more cycles of PCR using the labelled second set of primers and then subjected to binding to the said substrate. This would be particularly useful where there is an abundance of target DNA and/or where the detection means is very sensitive.

The following abbreviations are used in the present specification:
- PCR: Polymerase chain reaction
- DNA: Deoxyribonucleic acid
- A: Adenine
- T: Thymine
- G: Guanine
- C: Cytosine
- GST: Glutathione-S-transferase
- ADA: Amplified DNA assay
- HIV: Human immunodeficiency virus
- TMB: Tetramethylbenzidine
- ABTS: 2.2'-azino-bis-(3-ethylbenzthiazoline-6-sulphonic acid)
- MTPBS: Mouse tonicity phosphate buffered saline
- RT: Room temperature
- PBS: Phosphate buffered saline

In the accompanying drawings:

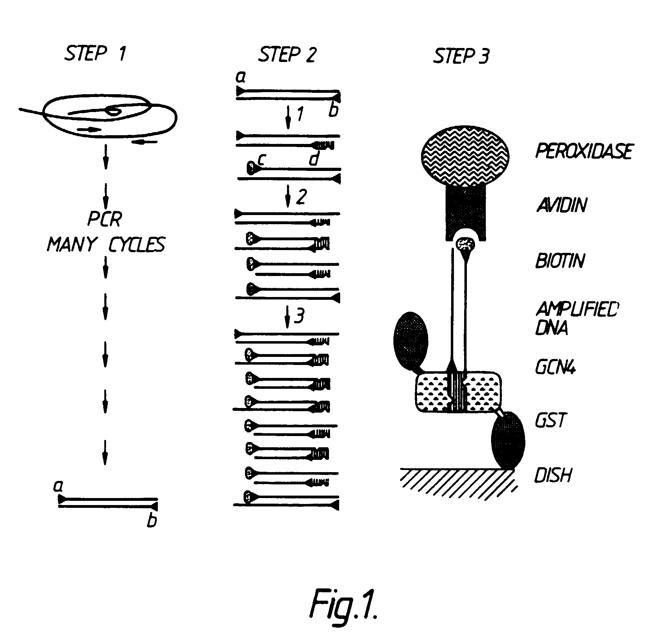

Figure 1 shows the three basic steps of one embodiment of the ADA. In step 1, a DNA segment is amplified from a biological sample via oligonucleotides a and b. In step 2, specific ligands are incorporated into the amplified DNA segment through at least 3 further cycles of amplification via internally nested oligonucleotides. Oligonucleotide c. bears a molecule of biotin at its 5' terminus and oligonucleotide d. bears a 5' nucleotide sequence specifically recognised by the DNA binding protein, GCN4, of Saccharomyces cerevisiae. In step 3, ligand bearing DNA segments are bound to a solid support coated with purified GCN4 produced in bacteria (GST-GCN4) and are detected via binding of avidin-peroxidase to the biotin of oligonucleotide c. and subsequent colorimetric detection of peroxidase activity.

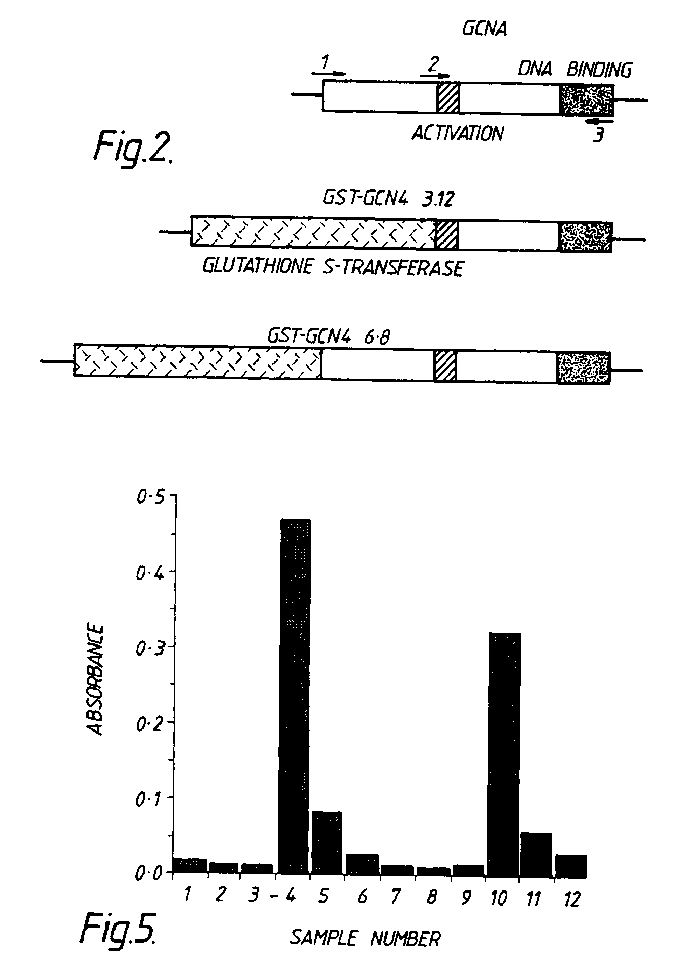

Figure 2 shows the structure of yeast GCN4 and GST-GCN4. At the top is the structure of the Saccharomyces cerevisiae GCN4 gene (9) with the coding region (281 amino acids) boxed and proposed transcription activation and DNA binding regions of the GCN4 protein indicated by hatching (10). Also indicated are the positions of oligonucleotides 1-3 used to amplify the GCN4 gene from yeast DNA by PCR. Fig.2 also shows the structure of genes encoding GST-GCN4 fusion proteins produced in E. coli by introducing fragments of the GCN4 gene into the plasmid expression vector pGEX-2T (11). The GCN4 gene was amplified from yeast DNA using oligonucleotides 2 and 3 or 1 and 3 to generate plasmids encoding partial (GST-GCN4 3.12) or full-length (GST-GCN4 6.8) versions of the GCN4 polypeptide fused to Schistosoma japonicum glutathione-S-transferase (GST).

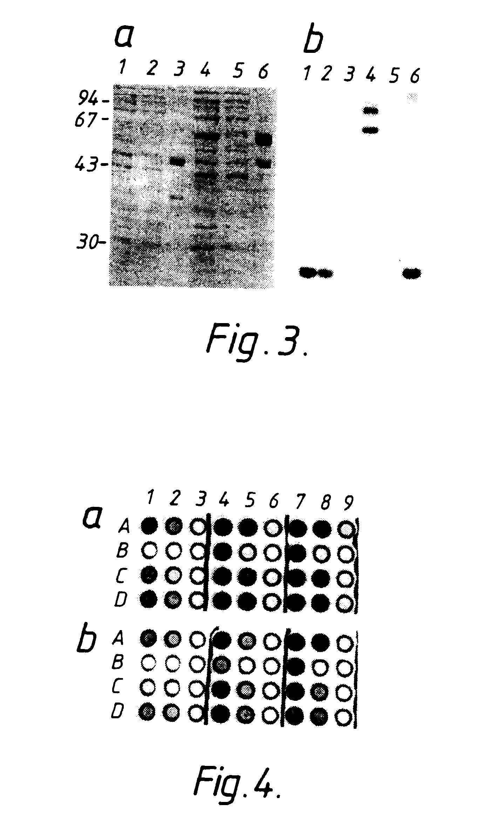

Figure 3a shows fractionation by polyacrylamide gel electrophoresis of total proteins (lanes 1 and 4) from E. coli strain 7118 transfected with plasmids pGST-GCN4 3.12 (lanes 1-3) or pGST-GCN4 6.8 (lanes 4-6) and grown in the presence of 0.1 mM IPTG for 1 hour at 37°C. Also shown is material purified from lysed bacteria by one-step affinity chromatography (lanes 3 and 6) and soluble proteins remaining after incubation with glutathione-agarose beads (lanes 2 and 5). Figure 3b shows a gel retardation assay demonstrating that the mobility of a ³²P labelled DNA fragment containing a GCN4 binding site is decreased when it is mixed with purified GST-GCN4 3.12 (lanes 2 and 4) or GST-GCN4 6.8 (lane 6) in comparison to its mobility in the absence of protein (lane 1).
Figure 4 shows the effect of added carrier DNA on the specificity of ADAs on DNA from HIV plasmid pHXBc2. In row A there was no added carrier DNA in the microtitre dish, while rows B, C and D contained 1µg/ml, 0.1 µg/ml and 0.01 µg/ml of sonicated human DNA, respectively. Step 1 of the ADA was for 30 cycles as described in Example 1, below, using oligonucleotides a. and b. and a Sac1 - Sal1 fragment from pHXBc2 (12). Samples (5ml) from step 1 were then amplified for a further 10 cycles using oligonucleotides c. plus b. (columns 1-3), c. plus d. (columns 4-6) or c. plus d2 (columns 7-9). Samples (5µl in 1, 4 and 7; 0.5µl in 2, 5 and 8; 0.05µl in 3, 6 and 9) were then added to wells of plates coated with purified GST-GCN4 3.12 (a) or GST-GCN4 6.8 (b). The remaining steps of the ADAs were as in Example 1.

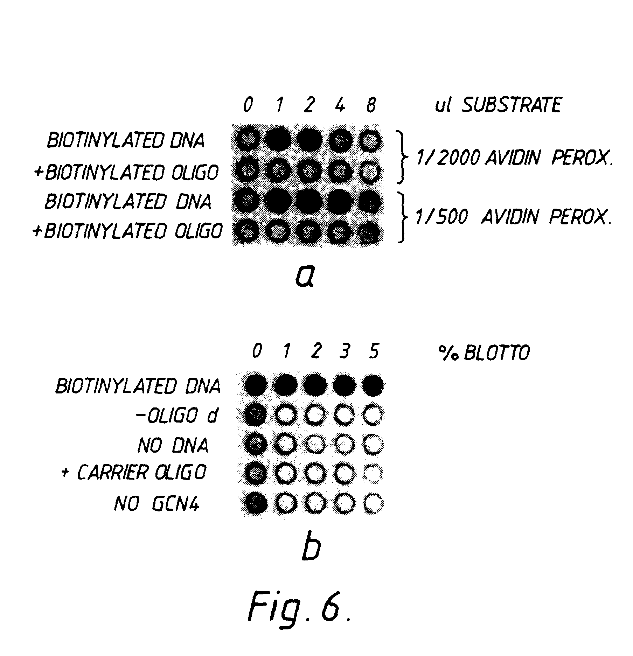

Figure 5 shows ADAs on DNA from HIV-infected cells. Samples 1-3, human DNA (^{~}100ng) from a Burkitt's lymphoma; samples 4-6, human DNA (^{~}100ng) from HIV-infected cells; samples 7-9, no DNA; samples 10-12, DNA (^{~}1ng) from plasmid pHXBc2. Step 1 of the ADA was for 35 cycles as described in Example 1 using oligonucleotides a. and b. Samples of 10µl from step 1 were then amplified a further 6 cycles using oligonucleotides c. plus d. Samples (15µl for 1, 4, 7 and 10; 3µl for 2, 5, 8 and 11; and 0.6µl for 3, 6, 9 and 12) were added to wells of plates coated with purified GST-GCN4 3.12 in the presence of sonicated human DNA (1µg/ml). The remaining steps of the ADAs were as in Example 1.
Figure 6A shows the specificity of the one step ADA reaction. Competition of unreacted biotinylated oligonucleotides with the ADA substrate in a one step binding reaction. A PCR was performed using oligonucleotides cl and dl (0.2µg) with 1ng of plasmid pHXBc2 in a 100µl reaction mix, cycled 24 times. The control did not contain any plasmid DNA. For rows 1 and 3 the volumes of PCR reaction indicated were added to 50µl of binding mix (without powdered milk) containing the dilutions of a 5 mg/ml avidin-peroxidase solution indicated. For rows 2 and 4, the volumes of PCR reaction indicated were added, and a further 8µl of the control PCR was added to each well. The binding reactions and colour development are described in Example 3.
Figure 6B shows the specificity of the one step binding reaction. The procedure was as for Fig. 6A, except that the wells contained the %(w/v) of non-fat powdered milk indicated. The top and bottom rows contained 5µl/well of the PCR mix described above. For row 2, oligonucleotide d was omitted. For row 3, there was no DNA in the PCR. Row 4 was as for row 3, but an unrelated oligonucleotide (1µg/ml) was added. For row 5, the wells were not coated with GCN4.

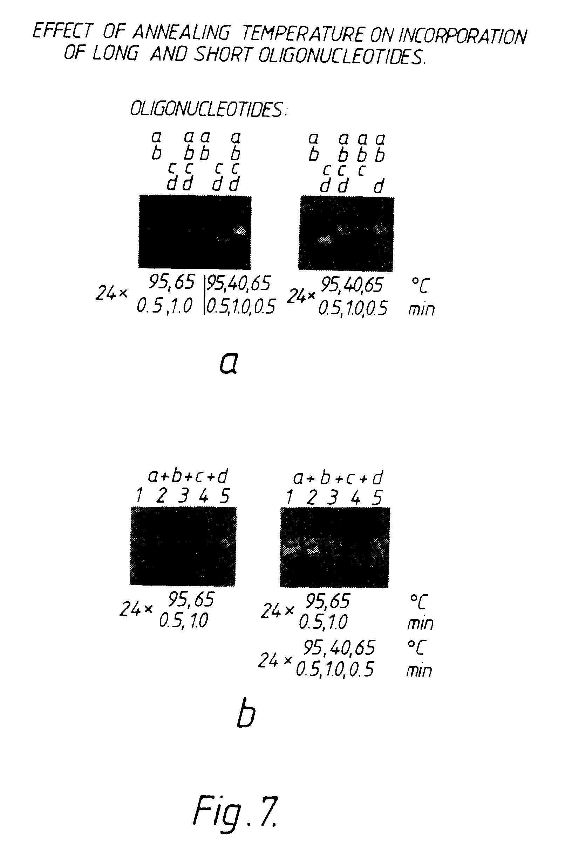

Figure 7 shows the effect of annealing temperature on incorporation. PCRs were carried out with 1ng plasmid pHXBc2 and oligonucleotides al and bl (1µg/ml), c2 and d2 (2µg/ml) as indicated, and cycled under the conditions shown, 10µl samples were fractionated on a 1.6% (w/v) agarose gel in the presence of 1µg/ml ethidium bromide. For A the concentrations of a1 and b1 were 0.3µg/ml. For B the concentrations of oligonucleotides a1 and c1 in the PCR were the following in successive tracks: a1= 6, 6, 2, 0.6, 2 µg/ml, b1= 3 µg/ml, c1= 20, 30, 10, 10, 20 µg/ml, d1= 5 µg/ml.

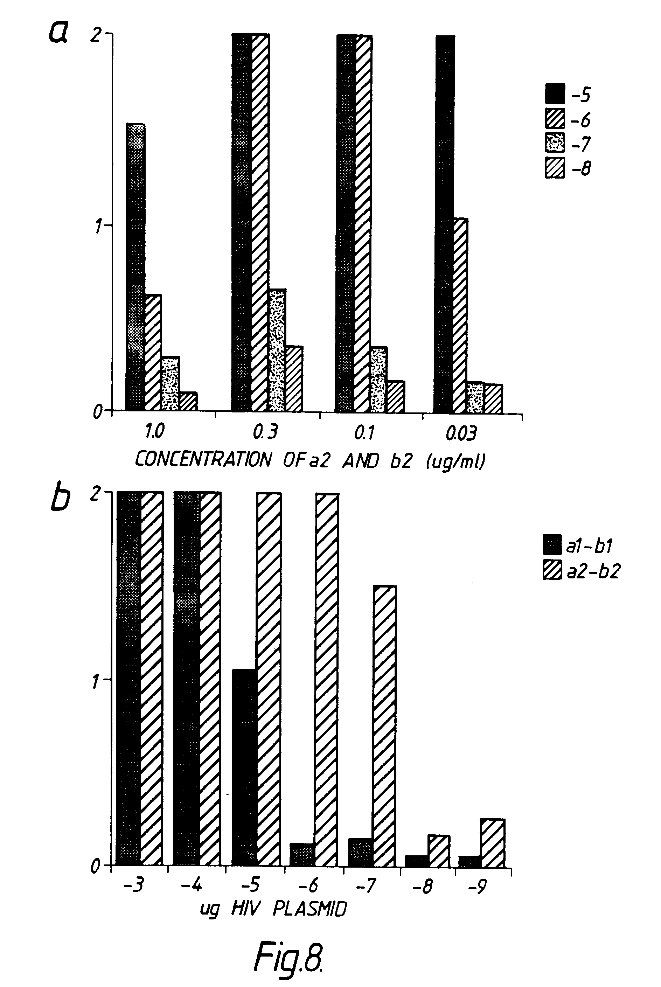

Figure 8A shows the sensitivity with different oligonucleotide concentrations. Two step PCRs were carried out with oligonucleotides a2 and b2 (concentrations as indicated) and c2 and d2 (5µg/ml) with the amounts of Plasmid indicated (at the right in µg) in a 20µl reaction, cycled 30 times (95°/30 sec, 65°/60 sec) and then 12 times (95°/30 sec, 40°/60 sec, 65°/30 sec). 5µl of the product was then analysed in an ADA with a one-step binding reaction.
Figure 8B shows the sensitivity of ADA reactions. Comparison of the sensitivity of ADA reactions using a and b oligonucleotides with different spacings from c2 and d2. The a and b oligonucleotides were at 0.3µg/ml.

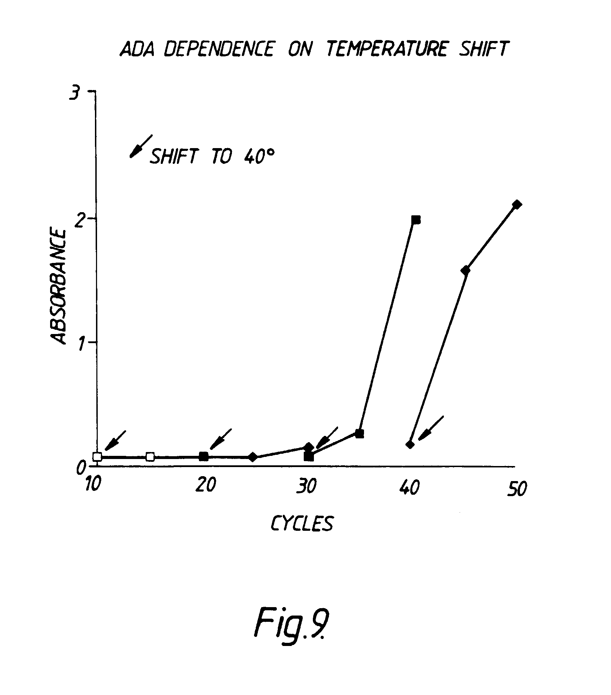

Figure 9 shows the ADA dependence on temperature shift. Two step PCRs were carried out with oligonucleotides a2, b2, c2 and d2 as in Fig. 3B. The DNA was from 5 x 10³ HIV-infected CEM cells. The PCRs were cycled at 95°/30 sec, 65°/60 sec for the number of cycles indicated by the arrows, and then at 95°/30 sec, 40°/60 sec, 65°/30 sec for 0, 5 and 10 further cycles. The number of cycles indicated at the bottom is the total number for each sample.

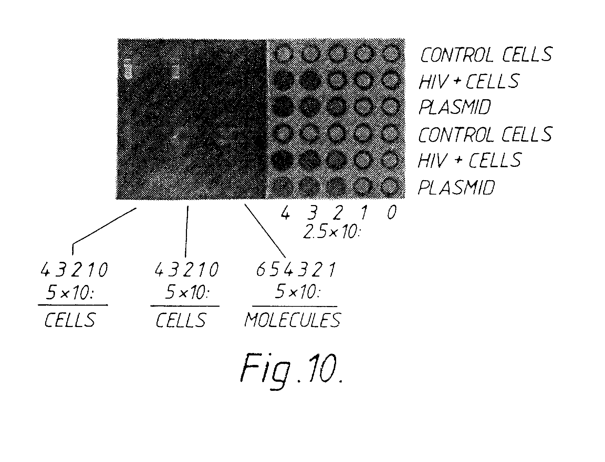

Figure 10 shows the detection of HIV in cultured cells. DNA from uninfected or HIV-infected CEM cells was used as the input DNA for PCR reactions containing oligonucleotides a2 and b2 (0.3µg/ml) and c2 (2.5µg/ml) and d2 (5µg/ml) that were cycled 30 times (95°/30 sec, 65°/60 sec) followed by a further 10 (top 3 rows in right panel) or 15 (bottom 3 rows in right panel, and left panel) cycles (95°/30 sec, 40°/60 sec, 65°/30 sec). Plasmid DNA was used as a positive control. ADA reactions with a one-step binding reaction were carried out on 5µl samples, and agarose gel electrophoresis on 10ml samples. The DNA samples analysed in the ADAs or by gel electrophoresis represented the material obtained from the number of cells indicated, or from the number of plasmid molecules indicated.

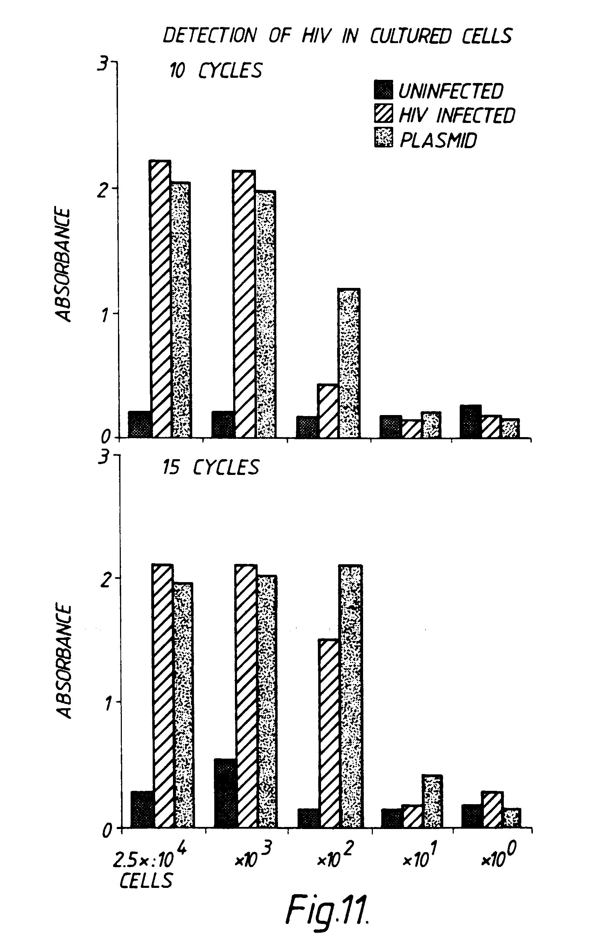

Figure 11 shows the quantitation of the ADA reactions shown in Figure 10.

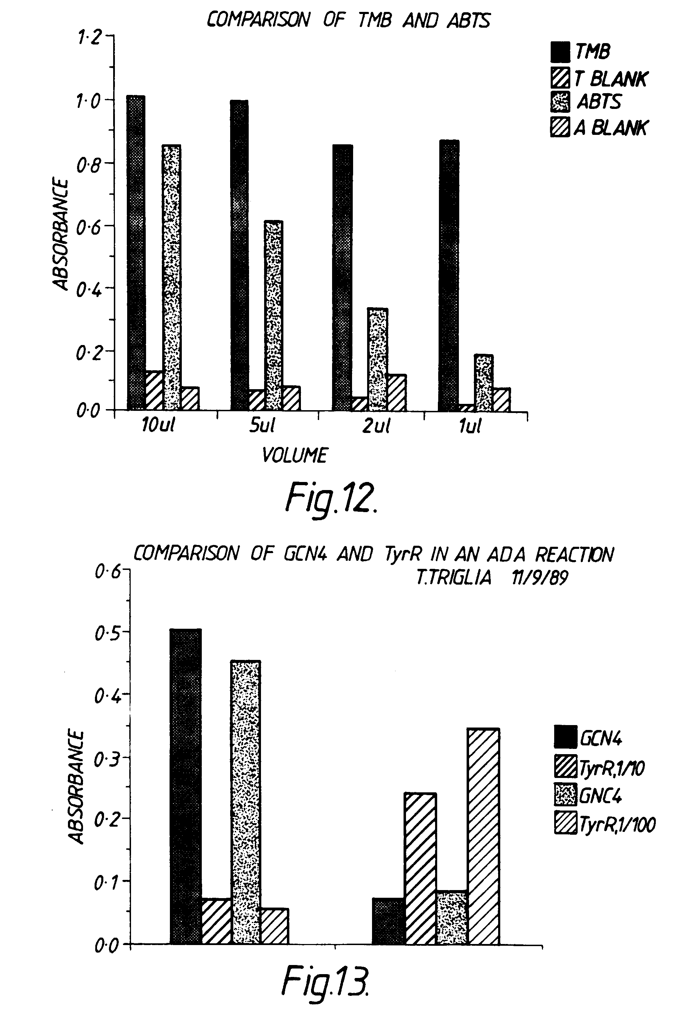

Figure 12 shows the comparison of TMB and ABTS in an ADA mediated by GCN4-coated pins.
Figure 13 shows a comparison of GCN4 and TyrR in an ADA reaction.

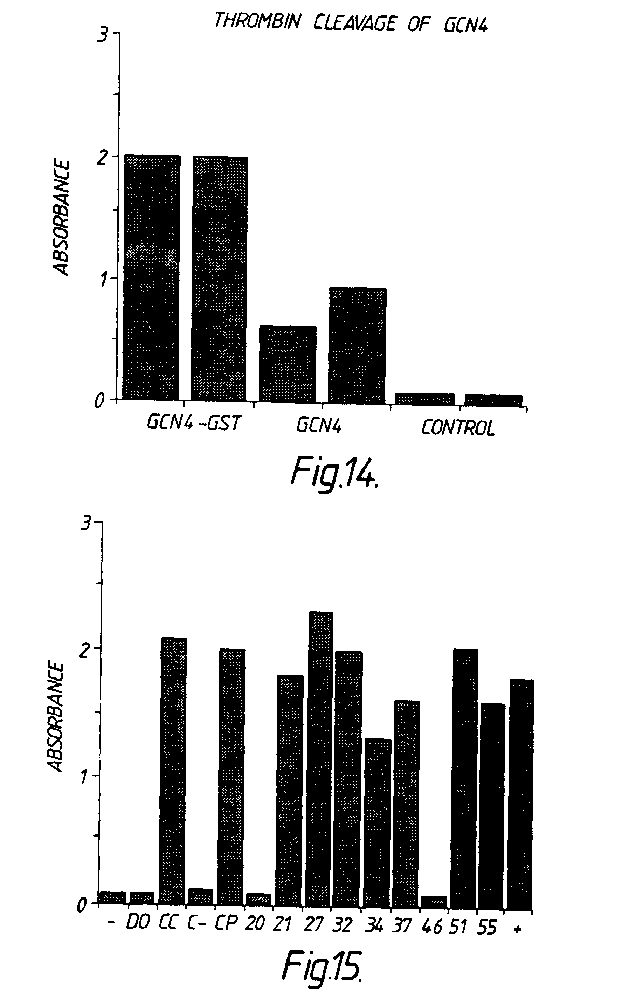

Figure 14 shows the effect of thrombin cleavage of GCN4 in the ADA.
Figure 15 shows the results of a clinical trial performed on Peripheral Blood Lymphocytes (PBL's) taken from patients positively diagnosed as suffering from AIDS or from negative controls. The method is as described in Figure 10 except 2 PCR's, 35 and 12 cycles respectively were used. Abbreviations are defined in Example 9.

In general terms, in one embodiment of the present invention, target DNA is first amplified by PCR using a first set of appropriate oligonucleotide primers in accordance with the known PCR procedure and then a second set of oligonucleotide primers, nested between the first two, are incorporated by a small number of additional cycles. The nucleotides in the second set of primers bear ligands; one can, for example, be biotinylated, and the other contains a site for a double stranded DNA binding protein. After linking to an immobilised affinity reagent, such as a DNA binding protein and labelling with a second affinity reagent, for example avidin linked to horseradish peroxidase, reaction with a chromogenic substrate allows detection of the amplified DNA. Furthermore, a system such as digoxigenin could be employed. Where there is sufficient target DNA without a first amplification or for other reason such as convenience or speed of assay, the target DNA may be directly subjected to incorporation by the labelled primers.

The assay procedure of the present invention is described in detail herein with particular reference to an assay for the detection of Human Immunodeficiency Virus (HIV) sequences. It will be understood, however, that this particular assay is described by way of exemplification of the invention and the invention has wider application as discussed below. Accordingly, by "target DNA" is meant any eukaryotic, prokaryotic or viral nucleic acid sequence and includes the identification of pathogens or the screening of human or other mammalian genetic disorders such as in cancer cells. Furthermore, target DNA encompasses RNA wherein by the action of reverse transcriptase, corresponding DNA is first synthesised, i.e. cDNA copied by reverse transcriptase from RNA. Target DNA also extends, therefore, to RNA viruses. Target DNA also extends to plant genetic sequences and to their pathogens.

Furthermore, the source of target DNA may vary depending on the particular circumstances and relative convenience. For example, one embodiment of the subject invention is described in terms of detecting HIV sequences in blood. However, this is done with the understanding these and other target sequences may be isolated from other bodily fluids such as, but not limited to, saliva. Accordingly, the present invention extends to the detection of target DNA in any suitable biological fluid such as blood, saliva, lymph fluid, cell and tissue extracts, culture supernatants, plant sap and/or other fluids or tissue extracts, aerosols, various environmental locations (eg soil, water, etc.) and the like.

The ADA procedure of this invention provides a very sensitive, specific, simple and convenient method for detecting specific DNA segments amplified by at least one PCR. The sensitivity of the method results from the combination of the inherent sensitivity of the PCR procedure itself (it can detect a single DNA molecule against a background of at least 10⁶ human genomes (2)) and a sensitive novel method for detecting the amplified DNA. The data below show that molecules of the ligand-containing amplified DNA can readily be detected and only a small fraction of the product from a typical PCR reaction is necessary for detection.

The specificity of the procedure reflects the fact that the ADA, in one embodiment, uses two successive PCR reactions with nested oligonucleotide primers. Only DNA molecules generated in the second step are detected in the final step because the ligands are only introduced during the second step. Alternatively, the target DNA may undergo the binding step directly without need of a first PCR. This specificity may be further increased, as in Example 2 herein, by the fact that GST-GCN4 only binds to double-stranded DNA - it does not recognise the single-stranded oligonucleotides. As the second step utilises only a small number of cycles (for example 3-12 cycles), there is insufficient time for accumulation of significant amounts of primer-dimers derived from the oligonucleotides of the second set or other double stranded DNA artefacts. Furthermore, any such artefacts generated in the first PCR step, for example by spurious priming at other places in the genome, are not amplified in the second step because they will not contain the nested sequences of the second set of primers. Primer-dimers formed in the first step will not be detected as they do not contain the ligands.

It is another embodiment of the subject invention that the PCR can be performed in one reaction mixture effectively resulting in a "single step ADA". This modification to the multi-step procedure previously outlined is predicated in part on a strong dependence of the thermal stability of an oligonucleotide duplex on its length and hence, oligonucleotide primers can be selected such that their incorporation in a PCR is critically dependent on the annealing temperature. Consequently, if one set of primers is considerably longer than a second set, then successive PCR reactions can be carried out in the one reaction mixture by incubating the mixture through first a high temperature and then a low temperature thermal cycle regime. (See Example 5).

The present invention, therefore, extends to both the multi-step and single step ADA.

The one step ADA also has advantages in the binding step where the binding of the amplified product to the double-stranded DNA-binding protein immobilized to a solid substance occurs simultaneously to the binding of or to a detection complex. In one embodiment, the amplified DNA is bound to GST-GCN4 immobilized in the wells of a microtitre dish while simultaneously binding to the avidin-peroxidase conjugate.

A further aspect of the one step ADA relates to the use of single or multiple beads or pins coated with a double-stranded DNA-binding protein to transfer the amplified product from a reaction vessel, after washing and contacting immobilized amplified product to a detection complex, to detection substrate. For example, the amplified DNA is transferred from a microtiter well by an array of GST-GCN4-coated beads or pins and, after washing and contacting with avidin-peroxidase, the beads are immersed in a microtiter dish containing ABTS substrate.

The successive PCR reactions can be carried out in the one reaction mixture, simply by incubating the mixture through first a high temperature and then a low temperature thermal cycle regime. As the complete PCR mixture containing all 4 oligonucleotides and enzyme (minus sample DNA) for this can be stored frozen, the protocol becomes greatly simplified, namely (1) the DNA sample is added to the PCR mixture, a drop of paraffin oil is added and the tube is placed on a thermal cycler and subjected to the two successive thermal regimes; (2) a sample is then placed in the GST-GCN4-coated microtiter well for simultaneous immobilization and binding; and (3) the dish is then washed and substrate added. This protocol is well suited to handle moderate numbers of samples. For example, the results for 50 samples can be obtained about 1 hour after completion of the PCRs.

In the one step assay, the amplified DNA binds to GST-GCN4 immobilized in the wells of a microtiter dish while simultaneously binding to the avidin-peroxidase conjugate. This decreases both the number of manipulations required and the time taken in handling samples, with no decrease in sensitivity or specificity. However, unincorporated biotinylated oligonucleotides compete with the amplified DNA for binding to avidin and it is necessary to ensure that the amount of biotin does not exceed the binding capacity of the avidin.

Furthermore, the one step assay also provides a protocol where the PCR with two successive thermal regimes is itself performed in a modified microtiter dish. The amplified DNA molecules are then bound to GCN4 immobilized on polystyrene beads attached to the lid of a microtiter dish. While this procedure cannot take advantage of the simultaneous immobilization and avidin-peroxidase binding, it has the very considerable advantage that after pipetting individual DNA samples into the first microtiter well, 96 samples can be handled simultaneously in a manner analogous to the widely used "FAST ELISA" system.

In all of these systems, streamlining of the detection systems has now reached the point where the time spent on preparing and handling individual DNA samples is the rate limiting event. The less abundant the target sequence, the higher the degree of purification that will be necessary. If the target sequence is detectable in less than 1µl of whole blood, then boiling the sample can be sufficient. However, if the required sensitivity demands that the total DNA from a large volume of blood is added to a single PCR, purification is required. It is self evident that this depends both in the intrinsic sensitivity of the assay (i.e., the number of relevant molecules than can be detected in an ideal situation) and the maximum amount of sample before inhibition of the system occurs. Blood seems to be a particularly bad DNA source because of the high protein content. Clearly, the minimal purification protocol necessary for a particular system is dependent on these parameters. Additionally, these modifications to the multi-step protocol described herein are capable of detecting HIV sequences against a background of human DNA.

The simplicity and convenience of the ADA results from the fact that after the PCR steps, the sample may be treated in precisely the same manner as a routine enzyme-linked immunosorbent assay (ELISA), using the same equipment. As the immobilisation by affinity binding to the solid phase (for example, GST-GCN4 or avidin) can be carried out in the same step as labelling at the other end of the DNA molecule (in the example, with avidin-peroxidase), the number of pipettings and washing is minimised. Further, if the solid phase coated with the affinity reagent consisted of pins in the roof of a microtitre dish, washing could be simplified. This latter approach also could readily lend itself to automation of the detection steps. The reactions in the example herein are extremely rapid because of the high affinities of avidin and GCN4 for their substrates, and the high Vmax of horseradish peroxidase.

The ADA system described in detail herein is only one possible formulation that has many alternatives. Obviously, the approach could be used for detection of many other viral, bacterial, protozoan, fungal and mycoplasmal pathogens. Screening for hepatitis, tuberculosis, malaria and candida infections are among the obvious applications involving these disparate organisms. Similarly, this system could be used for the detection of cellular disorders such as cancers and the like. The outstandingly useful feature of the ADA approach is that it is only necessary to change the sequences of the oligonucleotides in order to detect any gene from any organism by a simple colour test. If the length of the test DNA segment for each case is the same, then the kinetics of the detection steps should be identical as the same affinity reagents are interacting with the same ligands in all cases. This contrasts with the ELISA system where the affinity and kinetics are determined by the monoclonal antibodies, which differ for each situation. Another potential application lies in determining the genotypes of certain pathogens. For example, in Plasmodium falciparum, some genes contain variable regions defining different antigenic determinants surrounded by relatively conserved regions (13). If the probes of the first set of primers corresponded to such flanking conserved regions, the products of the first PCR step could be tested with several pairs of oligonucleotides corresponding to internal variable regions that define the different serotypes. The ADA described herein is also applicable to screening for genetic diseases such as cystic fibrosis and cancers amongst others.

The ADA system of the present invention could in theory employ a wide variety of ligands and/or affinity reagents. In one embodiment, the double-stranded DNA - specific DNA binding protein is of the leucine zipper type i.e. GCN4. A range of other DNA binding proteins of this type could be used, including thrombin-cleaved GCN4 (Figure 2, 14). Accordingly, the present invention extends to leucine zipper type DNA binding proteins such as GCN4 and/or its derivatives which includes GST-GCN4, thrombin-cleaved GCN4 and any other modifications thereof such as additions, deletion and/or substitution to the GCN4 amino acid and/or carbohydrate moieties provided said derivatives retain DNA binding activity

Another DNA binding protein that could be used in the ADA is the TyrR protein of the "Helix turn Helix" type and which has a C-terminal DNA binding domain (Dr V. Argyropolous, Thesis submitted for degree of Doctor of Philosophy, The University of Melbourne, Parkville, Victoria, Australia). Other DNA binding proteins which may be used in the ADA are well known and include, for example, the "zinc finger" type. These binding proteins are reviewed by Struhl (19).

As well as the applications to pathogens such as HIV used to develop the system here and the applications to genotyping pathogens mentioned above, the ADA system could be used with appropriate modification for virtually any application amenable to PCR itself (see for example references (1) to (8) and the Australian Patent Applications cited above). Major examples would include human genetics applications such as HLA typing and prenatal diagnosis of genetic disorders. The simplicity, specificity and generality of the approach should find many other applications.

Another embodiment of the subject invention employs a single PCR to incorporate directly label and/or ligand bound primers into target DNA without first being amplified and then exposing labelled target DNA to the solid support prior to, or simultaneously to, detection. This provides an even more streamlined method of detecting target DNA and makes the first PCR an optional step depending on the circumstances.

The present invention also extends to a conjugate consisting essentially of a support, GST-GCN4 immobilized on said support and an amplified double-stranded DNA bound at a first end to said GST-GCN4. The conjugate may further comprise at the second end of said double-stranded DNA, a label and preferably said label is an enzyme. In one embodiment the label is conjugated to the amplified DNA through an avidin-biotin bridge.

The present invention is further described by the following non-limiting examples.

### EXAMPLE 1

### MULTI-STEP ADA

### Materials and methods

### Isolation and expression of the GCN4 gene from S. cerevisiae.

The complete coding region of the GCN4 gene from S. cerevisiae (9) was synthesized by PCR on a crude DNA preparation using oligo 1 (GGAATTCTAATGTCCGAATATCAGCCA) and oligo 3 (GGAATTCAGCGTTCGCCAACTAATTTC) of GCN4, and incorporating EcoR1 sites at their 5' terminii (Fig.2). After cleaving with EcoRl, the DNA was ligated to EcoR1 cut DNA of the expression vector pGEX-2T (11). A smaller portion of GCN4 was also isolated by PCR using oligo 3 above and oligo 2 (CGGATCCATGTTTGAGTATGAAAACC) containing a BamH1 site at the 5' terminus (Fig.2) and insertion of the PCR product after cleavage with BamH1 and EcoR1 into BamH1 and EcoR1 cut pGEX-2T DNA.

### Gel retardation assay

Gel retardation was carried out as described (15) on 7.5% (w/v) polyacrylamide gels except that no blocking DNA was present. The substrate for binding was made by annealing 40ng each two oligonucleotides
(CCACCTAGCGGATGACTCATTTTTTTTCTTAGCG and
CGCTAAGAAAAAAAATGAGTC) and incubating them with Taq DNA polymerase (Cetus) in a reaction mix identical to that used for PCR except that dATP was replaced by 20mCi a³²P-dATP (Amersham). After 5 minutes at 70°C, dATP was added to 0.25mM and the incubation continued for 5 minutes. Unincorporated a³²P-dATP was removed by passage through a Sephadex G-10 (Trade Mark) spin column.

### Amplification of HIV Sequences - Step 1.

PCR reactions for amplification of p24 sequences from DNA isolated from HIV infected cells contained 50mM KCl, 10mM Tris pH 8.4, 2.5mM Mg Cl₂, 0.25mM each dATP, dCTP, dGTP and dTTP, 0.01% gelatin, 1.5 units Taq DNA polymerase (Cetus), 4ng oligonucleotide primers a. and b. and 100ng purified DNA. Reaction mixes (100ml) were cycled approximately 30 times between 40°C, 70°C and 95°C for 1.5, 2.0 and 1.5 minutes respectively.

### Incorporation of Ligands - Step 2.

One-tenth of a Step 1 PCR reaction was subjected to at least 3 additional cycles of PCR under identical conditions except that the primers used were oligonucleotides b. and c., c. and d. or c. and d1.

### Sequence of Oligonucleotides

The sequences (12) of the oligonucleotides corresponding to the p24 gene of HIV used were:

a.     AGAGAACCAAGGGGAAGTGA (positions 1481-1500)

b.     TCTCTAAAGGGTTCCTTTGG (positions 1661-1680)

c.	CATAGCAGGAACTACTAGTA (positions 1501-1520).

Oligonucleotide c. was biotinylated at the 5' end. d.    AAGTGACTCAAGTGACTCAA/TCCTTGTCTTATGTCCAGAA (nucleotides 5' to the slash correspond to an artificial GCN4 binding site (14), while those 3' to the slash correspond to positions 1641-1660). d1.    AGCGGATGACTCATTTTTTTT/TCCTTGTCTTATGTCCAGAA (nucleotides 5' to the slash correspond to an artificial GCN4 binding site (14), while those 3' to the slash correspond to positions 1641-1660).

### Preparation of DNA from HIV-infected cells.

CEM cells were derived by culturing human peripheral blood cells from a patient with acute lymphoblastic leukaemia, and then infected with HTLV IIIb. DNA was purified using guanidine HCl and CsCl centrifugation.

### Detection of Amplified DNA - Step 3.

Microtiter trays (Dynatech Laboratories Inc.) were coated with purified GST-GCN4 fusion polypeptides at approximately lµg/ml in mouse tonicity phosphate-buffered saline (MTPBS) for 3 hours at 37°C (50µl per well) and then blocked with 1% (w/v) bovine serum albumin (fraction V) (Flow Laboratories) in MTPBS for 1 hour at 37°C. Trays were then washed with MTPBS containing 0.05% (v/v) Tween-20, Trade Mark (MTPBS-Tw-20) and twice with MTPBS alone, before incubation at 20°C for 30 minutes with 50µl of ligand bearing DNA diluted in MTPBS-Tw-20. Trays were washed as before and incubated again at 20°C for 30 minutes with 50µl horseradish peroxidase -avidin D conjugate (Vector Laboratories, Inc.) at a concentration of 2.5µg/ml in MTPBS-Tw-20. After washing once in MTPBS-Tw-20 and four times in MTPBS, 100µl of fresh 0.1 M citrate pH 4.2 containing 1mM 2,2'-azino-bis(3-ethylbenzthiazoline-6-sulphonic acid) and 0.1% hydrogen peroxide were added to each well and absorbance read in a Titertek Multiskan MCC/340 (Trade Mark) scanner using filters of 414nm and 492nm.

### EXAMPLE 2

### Results of Multi-Step ADA

### a. The three basic steps of the ADA.

The approach to the detection of specifically amplified DNA is outlined in Figure 1. The ADA consists of three basic steps, the first two of which are different PCR reactions performed successively. A sample of the amplified DNA is then placed in a well of a microtitre dish for detection. As described above, there are many different possible permutations of the ADA of varying specificity and simplicity. For the sake of clarity, only one example of the theoretically most specific formulation is described in this section.

### Step 1: Amplification.

This step is simply a standard PCR reaction performed on any suitable DNA-containing extract relevant to the sequence of interest, for a large number of cycles. The oligonucleotides for step 1 (designated a and b in Figure 1) are limiting for this reaction. Step 1 simply amplifies the desired segment of DNA.

### Step 2: Sequence-specific ligand incorporation.

This step achieves specificity and simultaneously incorporates ligands into the PCR products that can react with affinity reagents, and thereby be detected in step 3. For step 2, two new oligonucleotides (designated c and d in Fig.1) are used for a second PCR reaction that can be cycled for as little as three cycles. This achieves specificity because oligonucleotides c and d are nested between oligonucleotides a and b. There are only a small number of cycles and therefore the only molecules that will form to a detectable extent are those generated by amplification of the correct sequence in step 1. Step 2 also incorporates the ligands. This can be done either as shown for oligonucleotide c, which is biotinylated, or as shown for oligonucleotide d which contains extra sequences encoding the recognition site for a double-stranded DNA binding protein, such as the yeast regulatory protein GCN4 (14). At least three cycles of step 2 are necessary to generate blunt-ended molecules with these ligands at both ends (Fig.1).

### Step 3: Anchoring and enzyme-linked-labelling of the amplified DNA by affinity binding.

This step attaches the amplified DNA to a solid phase by affinity binding to a double-stranded DNA-binding protein at one end, and is followed after washing by attachment of an enzyme by affinity binding at the other end for subsequent colour generation. For step 3, a sample from step 2 is added to a well of a microtitre dish. The well has been pre-coated with the DNA-binding protein, for example a cloned fused polypeptide bearing DNA binding protein GCN4 (see below). This polypeptide specifically immobilises the amplified molecules because of its affinity for double stranded DNA containing the correct sequence, incorporated via oligonucleotide d. After washing, a solution of the other affinity reagent conjugated to an enzyme, for example avidin linked to horseradish peroxidase, is added. This binds to the biotin linked to oligonucleotide c.

After washing, a chromogenic substrate is added to the microtitre dish, allowed to develop and the absorbance is read in a microtitre-plate reader.

### b. Generation of a DNA binding protein that can be readily purified.

In order to generate large amounts of a high-affinity DNA binding protein suitable for routine use in the ADA, the Saccharomyces cerevisiae regulatory protein GCN4 has been expressed as a glutathione-S-transferase (GST) fusion protein as shown in Figure 2. Plasmid pGST-GCN4-3.12 contains most of the sequence of GCN4 from Saccharomyces cerevisiae, including the C-terminal DNA-binding region, inserted into the plasmid pGEX-2T (11), while the plasmid pGST-GCN4-6.8 contains the entire coding sequence of GCN4. At the N-terminus, the GST-GCN4 fused polypeptide contains the entire sequence of glutathione-S-transferase (GST) from Schistosoma japonicum, which allows purification of the molecule in one simple affinity step by binding to glutathione-agarose beads (11). Figure 3 shows that the GST-GCN4 polypeptides are abundant in Escherichia coli clones transformed with these plasmids. After one-step affinity purification, each of the GST-GCN4 polypeptides was detected as two Coomassie-blue stained bands after polyacrylamide gel electrophoresis (Fig.3a). These purified proteins retain the ability to bind to the consensus GCN4 binding sequence as revealed by a gel retardation assay (Fig.3b).

Hence both of the necessary affinity reagents (GST-GCN4 and avidin) are now readily available.

### c. Application of the ADA to a DNA segment encoding a Human Immunodeficiency Virus (HIV) sequence.

Because of the important clinical implications a region of the HIV genome was chosen as test sequence. Oligonucleotides corresponding in position to those labelled a, b, c and d in Fig.1 were synthesised for the p24 gene of HIV and are defined in Materials and Methods. To develop the ADA, plasmid pHXBc2 bearing this gene was used initially as the test source. In initial studies to establish that the affinity reactions were feasible, DNA molecules with a biotin ligand at one end and a GCN4 binding site at the other were generated by PCR amplification of the plasmid with oligonucleotides a. and b. followed by amplification with oligonucleotides c and d. After binding these products to a microtitre well precoated with GST-GCN4 followed by washing and binding of avidin linked to peroxidase, and then washing and reaction with the chromogenic substrate, an intense reaction was observed (Fig.4). This was not observed when the PCR was carried out in the absence of plasmid DNA (Fig.5, samples 7-9).

When the amplified DNA was added to the microtitre dish in the absence of carrier DNA, the GST-GCN4 was found to bind to double-stranded DNA independently of a GCN4 binding sequence. This can be seen in row A in Fig.4 where oligonucleotides b. and c. (which both lack a GCN4 binding site) were used in the second step. However, this product formed with oligonucleotides b. and c. did not bind in the presence of carrier DNA (row B in Fig.4, columns 1-3) whereas the corresponding products formed with oligonucleotides c. and d. (row B in Fig.4, columns 4-6) or c. and d2 (row B in Fig.4, columns 7-9) still bound, as indicated by the strong reaction, although this signal is lower than in row A. Intermediate levels of carrier DNA partially competed (rows C and D). It seems that GST-GCN4 3.12 and 6.8 have similar activities and specificities.

### d. Application of the ADA to human cells infected with HIV.

To examine whether the ADA could detect HIV sequences specifically in DNA from infected human cells, purified DNA from persistently infected cells was used. With DNA from uninfected cells, there was no detectable signal (Fig.5, samples 1-3) while a strong signal was obtained with DNA containing HIV (Fig.5, samples 4-6).

### EXAMPLE 3

### Single Step ADA

### Materials and Methods

### GST-GCN4

The fused polypeptide from clone GST-GCN4 3.12 (16) was purified by binding to glutathione-agarose as described (11).

### TyrR

TyrR a DNA binding protein of the "Helix turn Helix" type, and which has a C-terminal DNA binding domain was provided for testing by Dr V. Argyropolous (18).

### PCR reactions

PCR reactions for amplification of p24 sequences of HIV contained 50mM KCl, lOmM Tris pH 8.4, 2.5mM MgCl₂ 0.25mM each dNTP, Taq polymerase (0.5 unit) and oligonucleotide primers at various concentrations. Reaction mixes (20µl) were incubated under paraffin oil using the conditions described below. For routine use, the PCR mixes containing all components except DNA were stored as frozen aliquots.

### Amplified DNA assays

i) One step binding assays: Microtiter trays (Dynatech Laboratories Inc.) were coated with purified GST-GCN4 fusion polypeptides at approximately 5µg/ml of the active product(s) in phosphate buffered saline (PBS) for 1 hr at 37°C, washed 1X and then blocked with 10% (w/v) non-fat powdered milk in PBS. The plates were then drained, but not washed, and 50µl/well of a mixture containing 10% (w/v) non-fat powdered milk in PBS, 4µg/ml sonicated salmon DNA, 0.05% (v/v) Tween-20 and 50µg/ml horseradish peroxidase-avidin D conjugate (Vector Laboratories Inc.) in PBS was added. Samples of the PCR reactions (1-10µl) were then added and allowed to react for at least 20 min at RT. Trays were washed with MTPBS-Tween-20 four times, with MTPBS four times, with H₂O once, drained and then 100ml of fresh 0.1M Na citrate, pH 4.2, containing 1mM 2.2'-azino-bis (3-ethylbenzthiazoline-6-sulphonic acid) (ABTS) and 0.1% (v/v) hydrogen peroxide were added to each well and absorbance read in a Titertek Multiskan MCC/340 scanner on mode 2 using filters of 414nm and 492nm.

### ii) ADAs with GST-GCN4 immobilized on beads.

The beads on the lid of a "FAST ELISA" dish (Falcon plastics) with the corners cut off were coated with GST-GCN4 by placing them in 50µl aliquots of GST-GCN4-PBS in a microtiter tray (Dynatech Laboratories Inc.) as above for 1 hr at 37°C, and then blocked in a solution containing 10% (w/v) non-fat powdered milk, 4µg/ml salmon DNA and 0.05% (v/v) Tween-20 in MTPBS. The lid was then flicked to drain off excess solution and the beads placed in the microtiter dish containing the PCR samples. After 20 min at RT, the beads were washed with PBS-0.05% (v/v) Tween 20. They were then reacted with 10µg/ml peroxidase-avidin conjugate in 10% (w/v) powdered milk, 0.05% (v/v) Tween-20 in MTPBS for 20 min. They were then washed extensively with PBS and reacted with ABTS as above. Alternatively, they were reacted with 0.4mM Tetramethylbenzidine (TMB) in 0.1M NaAc, pH 5.5 plus 1.41mM hydrogen peroxide and read in a Titertek Multiskan MCC/340 scanner on mode 1 using filter number 7.

### Oligonucleotides

Consensus oligonucleotides corresponding to sequences from the gag gene of HIV were selected after aligning available sequences from the HIV database. The oligonucleotides synthesized were:

al ATGAGAGAACCAAGGGAAG
(1470->1489)

a2 GGGGGACATCAAGCAGCCATGCAAATG
(1362->1388)

b1 TTGGTCCTTGTCTTATGTCCAGAATGC
(1656<-1630)

b2 ACTCCCTGACATGCTGTCATCATTTCTTC
(1846<-1818)

cl 5'Biotin-CATAGCAGGAACTACTAGTA
(1493->1512)

c2 5'Biotin-CAGGAACTACTAGTA
(1498->1512)

d1 AAGTGACTCAAGTGACTCAATCCTTGTCTTATGTCCAGAA
(1652<-1633)

d2 GGATGACTCATAGGGCTATACATTC
(1625<-1611)

### DNA from HIV-infected cells

CEM cells were derived by culturing human peripheral blood cells from a patient with acute lymphoblastic leukaemia, and then infected with HIV isolate HTLV IIIb. DNA was purified using guanidine HC1 and CsC1 centrifugation.

### DNA from clinical blood samples

DNA was purified from peripheral blood leucocytes using guanidine HCl and phenol/chloroform/ethanol centrifugation.

### Plasmid DNA

Plasmid pHXBc2 (12) encoding the GAG gene of HIV was used as a source of DNA for developing the reactions. Generally, 1µl of a 1µg/ml solution per 1000ml of PCR was used.

### EXAMPLE 4

### A one-step binding reaction for the ADA

In the ADA described in Examples 1 and 2, the amplified DNA was first captured on GST-GCN4 immobilized in a microtiter well, the unincorporated substrates washed away and then avidin-peroxidase bound to the amplified DNA molecules. To simplify the procedure the amplified DNA was mixed with the avidin-peroxidase conjugate in the presence of protein and DNA carriers and these were bound to the immobilized GST-GCN4 in a single reaction mixture. Experiments with increasing amounts of the PCR sample and with PCR mix without DNA template showed that unincorporated biotinylated oligonucleotide rapidly competed out binding, as measured by the subsequent colour development after washing away the conjugate and adding substrate (Fig 6A). By increasing the concentration of avidin and decreasing the amount of biotin, this effect could readily be overcome. However, the increased level of peroxidase raised the background. This could be prevented by blocking protein binding sites with high levels of protein carrier, for example 10% (w/v) powdered milk, after coating the wells with GST-GCN4 (Fig 6B). Control experiments showed that it was essential to have GST-GCN4 on the plate and an appropriate target sequence on the amplified DNA (Fig 6B).

### EXAMPLE 5

### Thermal separation of the two PCR steps

Because there is a very strong dependence of the thermal stability of an oligonucleotide duplex on its length, it was anticipated that it would be possible to choose lengths for oligonucleotides a, b, c and d (see page 26) such that their incorporation in a PCR would be critically dependent on the annealing temperature. If oligonucleotides a and b are considerably longer than c and d so that they form duplexes that are considerably more stable than those of c and d, then annealing at a sufficiently high temperature should prevent incorporation of c and d, allowing separation of the reactions in a mixture containing all four oligonucleotides. Preliminary studies showed unexpectedly that when different oligonucleotides 18-20 bases long were used, annealing temperatures as high as 70°C did not prevent incorporation, although the efficiency was reduced. However, when c and that part of d complementary to the HIV sequence were 15 bases long, a clear thermal separation could be obtained at 65°C.

Hence, with a thermal cycling regime of only two steps per cycle, 95°C for 1 min followed by 65°C for 2 min (termed "without annealing") oligonucleotides al and bl (20 and 28 bases long respectively) were incorporated in 24 cycles using lng of plasmid DNA as the template but there was no incorporation of c2 and d2 (Fig 7A, left panel) even with this high template input. If an annealing step of 40°C for 1 min was introduced ("with annealing") as well as the other 2 steps, c2 and d2 could be efficiently incorporated in an additional 24 cycles (Fig 7A, left panel). Similarly, with a total of 24 cycles all with annealing c and d were efficiently incorporated (Fig 7A, right panel). With annealing incorporation of al and bl was less efficient (Fig 7A, right panel).

When all four oligonucleotides were included (Fig 7A & B), only al and bl were incorporated when cycled without annealing as expected. Surprisingly, however, with annealing the expected c2-d2 product was not obtained. Instead, a longer product corresponding to either a1-d2 and/or c2-b1 was obtained. Hence, it appears that either c2 or d2 or both are competed out by a1 and/or b1. In the absence of c2, this product was obtained, but in the absence of d2, it was not indicating that it is an a1-d2 product (Fig 7A, right panel). Accordingly, the effect of lowering the relative amounts of a1 and b1 to c2 and d2 was investigated. Figure 7B shows that when the amounts of a1 and b1 were decreased sufficiently, the expected c2-d2 product could indeed be obtained when cycled with annealing. As expected, only the a1-b1 product was formed without annealing, and the amounts of this were not greatly affected by lowering the concentrations of a1 and b1 (Fig 7B, left panel).

ADAs on the products of these reactions were also performed (data not shown). In order for the amplified DNA to function in such an assay, it must contain a biotin moiety at one end and a GCN4 binding site at the other end. Only those reaction mixtures containing the short c-d product gave a significant colour reaction in the ADAs, confirming the structure of these molecules.

It is concluded that it is possible to separate PCR steps 1 and 2 thermally by choosing oligonucleotides of appropriate length. The products of the second reaction act as substrates in ADAs as expected. However, there is a competition effect that can readily eliminate the reaction if the concentrations of oligonucleotides a and b are not carefully controlled.

Studies with a series of dilutions of the HIV plasmid, cycled for various times under the two different temperature regimes revealed some further features of the reactions. First, it was clear that the colour intensity depended on the number of cycles both with and without annealing. However, under these conditions the sensitivity is limited: at least 10⁴ molecules are required (Example 6 below). Nevertheless, it is sensitive enough to detect HIV sequences in human DNA from persistently infected cells while the uninfected control was negative.

### EXAMPLE 6

### Thermal separation of the two PCRS, using more widely spaced oligonucleotides.

The apparent competition of the a and b oligonucleotides with the c and d oligonucleotides (Example 5) could result in part from steric hindrance and from kinetic effects related to the rates of annealing of the oligonucleotides. It has been noted previously (16) that this may be exacerbated by close spacing of a/c and b/d. Alternatively, c and d oligonucleotides that anneal to and are extended on the a-b template could subsequently be removed by nick translation after a second initiation event with an a or b oligonucleotide on the same template molecule in the same extension cycle. It is now clear that Taq polymerase has a 5'->3' exonuclease activity and so it can translate nicks. These effects should all be lowered if the spacing of oligonucleotides a/c and b/d are increased. Accordingly, oligonucleotides a2 and b2 were synthesized corresponding to conserved positions considerably further away from c2 and d2 than are al and b1.

After cycling first without and then with annealing, incorporation of oligonucleotides c2 and d2 was dependent on the concentration of oligonucleotides a2 and b2 (Fig 8a). At the optimal concentrations of a2 and b2, the c2-d2 product could be detected either by an ADA reaction or by EtBr staining from about 100 fold less input plasmid DNA than with oligonucleotides a1 and b1 (Fig 8b). Production of the c2-d2 product and colour intensity in an ADA was dependent on the number of cycles both without and with annealing and there was no significant c2-d2 product without annealing, even after 40 cycles (Fig 9).

Under these conditions, HIV sequences could be detected in the DNA obtained from about 250 cells from an HIV-infected CEM culture while there was no significant background even with 100-fold more DNA from uninfected cells (Figs 10 and 11). It can be seen on the gel shown in Fig 10 that the two successive reactions with nested oligonucleotides are indeed vital to the specificity - there are many bands generated from uninfected DNA, but these do not register as positive in the ADA.

Hence, the use of a and b oligonucleotides located further out from c and d considerably increased the sensitivity with no loss of specificity.

### EXAMPLE 7

### Development of a system for assaying PCR reactions performed in microtiter dishes

One way to further simplify the ADA system is to perform the PCRs in a microtiter dish and then to capture and transfer the amplified DNA from each of the 96 wells to a second dish using an array of GST-GCN4-coated beads or pins. To test whether this was feasible, 1-10µl samples of a PCR reaction performed with oligonucleotides c2 and d2 were made up to 20µl in a microtiter dish and covered with a drop of paraffin oil. The beads of a FAST ELISA screening plate were coated with GST-GCN4, blocked with powdered milk-DNA and then immersed in the PCR samples for 20 min. Subsequently the beads were washed, exposed to avidin-peroxidase, washed and placed in a microtiter dish containing ABTS substrate. The responses obtained were proportional to the amount of amplified DNA (Fig 12), and there was negligible background from equal amounts of a PCR mix incubated without substrate DNA. The colour intensity was lower by a factor of 2-3 than reactions performed using GST-GCN4-coated wells with the same material (data not shown) reflecting the lower surface area of beads. However, the sensitivity could be increased approximately 10-fold using TMB as the substrate, without any significant increase in background (Fig 12). Hence, amplified DNA molecules can be captured and transferred efficiently using GCN4 coated beads. This is surprising given that the coated beads were first dipped through a layer of paraffin oil, mimicking the conditions necessary for a PCR.

To establish that PCR reactions could be performed in a microtiter dish and then transferred as above, reactions with oligonucleotides a1 and b1, c2 and d2 or all four oligonucleotides were set up as for Fig 2 and incubated in the wells of a flexible microtiter dish mounted on a hollow aluminum block through which water at the appropriate temperature was circulated. The top of the block was milled to fit the bottom of the dish and zinc oxide heat-sink cream was used to ensure thermal contact. Evaporation was prevented by a drop of paraffin oil. After 24 cycles with a 40°C annealing step, oligonucleotides a1-b1 and c2-d2 were incorporated into products of the expected size. Furthermore, the c2-d2 product gave an ADA reaction as expected (data not shown).

### EXAMPLE 8

### Use of another DNA binding protein, TyrR and thrombin-cleaved GCN4 in the ADA

It would be useful for some purposes to have other DNA binding proteins, with different DNA recognition sequences, that could work in an ADA reaction. For example, a set of HIV-oligonucleotides with a GST-GCN4 site could be included in the same mix as a set of hepatitis B viral oligonucleotides marked with a second DNA binding protein site, so each could be read specifically from the one PCR. TyrR is a DNA binding protein of the "Helix turn Helix" type, and which has a C-terminal DNA binding domain was provided for testing by Dr V. Argyropolous (18).

An oligonucleotide probe was manufactured which contained a TyrR recognition site and a HIV sequence, i.e. corresponding to the oligo "d" described in Example 3 except that the TyrR recognition site replaced the GCN4 binding site.

The probe was of the sequence

5'
TGTGTAAATATATATTTACACA/AGGGCTATACATTC
TyrR recognition site    HIV oligo

This probe was incorporated into the ADA test with the oligo "c" and reaction products tested on plates coated with GST-GCN4 or TyrR.

Results were compared with those of the same experiment but using the original oligo "d" containing the GST-GCN4 binding site as shown in Figure 13.

### EXAMPLE 9

### Clinical Trial

The following example shows the results of a clinical trial performed on Peripheral Blood Lymphocytes (PBL's) taken from patients positively diagnosed as suffering from AIDS or from negative controls. The trial was performed blind.

The PBL's were prepared from blood samples by lysis in Guanidine thiocyanate buffer (4M) and centrifugation.

DNA was extracted and purified from PBL's using the same technique of guanidine thiocyanate centrifugation.

One step ADA reactions were performed using oligos a2, b2, c2 and d2 as described in Example 3.

The legend for the results provided in Figure 15 is as follows:
- -: No DNA
- DO: Human DNA (negative control)
- CC: Positive control using CEM cells transfected with HIV
- CP: Positive control using Plasmid DNA
- +: Positive control using CEM cells having Plasmid DNA incorporated therein.
- 20-55: Clinical specimens

### Samples 20 and 46 were obtained from healthy humans.

The results clearly show the sensitivity and specificity of the ADA to detect HIV.

### EXAMPLE 10

### Detection of Mycoplasma pneumoniae using the ADA

This example shows that the ADA can be used to detect DNA from M. pneumoniae, a pathogen which can cause severe respiratory tract infections. The first experiment used plasmid DNA containing the P1 gene of M. pneumoniae as described in Hu et al, Gene, 64,217, and one pair of ADA primers. The second experiment used clinical specimens (nasopharyngeal aspirate) to which had been added whole M. pneumoniae cells, and two nested pairs of primers.
(i) This experiment used only one pair of ADA primers, one of them being biotinylated, and the other containing the GCN4 recognition sequence, as follows: 
ADA primer 1: 5'biotinyl - TCAAAACAACGACAC-3'
(corresponding to nucleotides 3863-3877 of the P1 gene)
 
ADA primer 2: 5'-GGATGACTCATTTCAGAAAGTCGAC-3'
(corresponding to nucleotides 4144-4100 of the P1 gene;
the first 10 nucleotides constitute the GCN4 site)
The ADA consisted of taking purified P1 plasmid DNA and adding 0.6 µmolar/120ng of each of ADA primers 1 and 2. The first 5 cycles of amplification were conducted as follows: 94°C - 1 minute (melting); 37°C - 2 minutes (annealing); 60°C - 3 minutes (extension). The next 25 cycles were: 90°C - 1 minute; 37°C - 2 minutes; 60°C - 3 minutes; with the final extension at 72°C for 10 minutes.
Approximately 1/5 of the product was then analysed in a one step binding reaction in which microtitre plates were coated with GST-GCN4 (250ng/well in PBS) overnight at 4°C. Detection of amplified DNA was performed as previously described. In the second part of this experiment, a smaller (i.e. 1/10) amount of DNA was used as starting material.
The mixture used for performing the PCR was:
2 units Taq polymerase (Cetus),
10mM Tris HCl pH 8.3,
50mM KCl,
1.5mM MgCl₂,
0.2% µmolar dNTP's,
plus ADA primers as above, in a volume of 50µl.

(ii) This experiment used 2 pairs of primers - the ADA primers described above and another pair, the PCR primers, which lie outside the ADA primers, as follows: 
PCR primer 1: 5'-CAAGCCAAACACGAGCTCCGGCC-3'
(corresponding to nucleotides 3666-3688 of the P1 gene)
 
PCR primer 2: 5'-CCAGTGTCAGCTGTTTGTCCTTCCCC-3'
(corresponding to nucleotides 4208-4183 of the P1 gene)
Various amounts of whole M. pneumoniae cells were added to nasopharyngeal aspirate and the amount of cells added measured as the approximate number of genomes. The material was centrifuged and the precipitate collected. This was treated with proteinase K (30µl, 200 µg/ml in 10mM Tris CL pH 8.3) at 37°C/1 hour to free the DNA, then at 95°C/15 minutes to inactivate the proteinase K. The ADA was then conducted as described below.
The PCR mixture used was as for experiment (i), except that it included 250ng of each ADA primer and 5ng of each PCR primer.
The first round of cycling (i.e. that which favoured the PCR primers) comprised 30 cycles as follows: 94°C - 1 minute; 65°C - 2 minutes; 72°C - 3 minutes. The second round, for the ADA primers, comprised 15 cycles as follows: 90°C - 1 minute; 40°C - 1 minute; 60°C - 3 minutes; followed by a final extension step at the end of 72°C for 10 minutes.
Analysis and detection of the amplified DNA was performed in the same manner as in experiment (i).
(iii) The results obtained in experiments (i) and (ii) are set out in the following tables (in which the results are scored using a plus(+) scale, with maximum colour being ++++ and no colour being -):

| Experiment (i) | |
|---|---|
| *Amount of plasmid DNA* | *Result* |
| | |
| 56ng (7x10⁸ molecules) P1 plasmid: | ++ |
| 5.6ng (6x10⁷ molecules) P1 plasmid: | + |
| No plasmid control | - |

| Experiment (ii) | |
|---|---|
| *Approx no. of M. pneumoniae genomes* | *Result* |
| 5x10⁸ | +++ |
| 2.5x10⁸ | + |
| 10⁸ | ++ |
| 10⁷ | +/- |
| 5x10⁵ | - |
| 2.5x10⁵ | - |
| 10⁵ | - |
| 10⁴ | - |
| 0 | - |

### REFERENCES:

1. Saiki, R.K., Scharf, S., Faloona, F., Mullis, K.B., Horn, G.T., Erlich, H.A. and Arnheim, N. (1985) Science 230, 1350-1354.
2. Saiki, R.K., Gelfand, D.H., Stoffel, S., Scharf,S.J., Higuchi, R., Horn, G.T., Mullis, K.B. and Erlich, H.A. (1987) Science 239, 487-491.
3. Lee, C.C., Wu, X., Gibbs, R.A., Cook, R.G., Muzny, D.M. and Caskey, C.T. (1988) Science 239, 1288-1291.
4. Gyllensten, U.B. and Erlich, H.A. (1988) Proc. Natl. Acad. Sci. USA 85, 7652-7656.
5. Triglia, T., Peterson, M.G. and Kemp, D.J. (1988) Nuc. Acids Res. 16, 8186.
6. Kim, H-S. and Smithies, O. (1988) Nuc.Acids Res. 16, 8887-8904.
7. Landegren, U., Kaiser, R., Caskey, C.T. and Hood, L. (1988) Science 242, 229-237.
8. Laure, F., Rouziox, C., Veber, F., Jacomet, C., Courgnaud, G., Blanche, S., Burgard, M., Griscelli, C. and Brechot, C. (1988) The Lancet, 538-541.
9. Hinnebusch, A.G. (1984) Proc. Natl. Acad. Sci. USA 81, 6442-6446.
10. Hope, I.A. and Struhl, K. (1986) Cell 46, 885-894.
11. Smith, D.B. and Johnson, K.S. (1988) Gene 67, 31-40.
12. Sodroski, J., Patarca, R., Rosen, C., Weng-Staah, F. and Haseltine, W. (1985) Science 229, 74-77; Sanchez-Pescadon, R., Power, M.D., Barr, P.J., Steinmer, K.S., Stempien, M.M., Brown-Shimer, S.L., Gee, W.W., Renard, A., Randolph, A., Levy, I.A., Dina, D. and Luciw, P.A. (1985) Science 227, 484-492.
13. Kemp, D.J., Coppel, R.L. and Anders, R.F. (1987) Ann. Rev. Microbiol. 41, 181-208.
14. Hill, D.E., Hope, I.A., Macke, J.P. and Struhl, K. (1986) Science 234, 451-457.
15. Hope, I.A. and Struhl, K. (1985) Cell 43, 177-188.
16. Kemp, D.J., Smith, D.B. Foote, S.J., Samaras, N. and Peterson, M.G. (1989). Proc. Natl. Acad. Sci. USA **86,** 2423-2427
17. Hinnebusch, A.G. (1984) Proc. Natl. Acad. Sci. USA **81,** 6442-6446.
18. Argyropolous, V. (1989) Thesis for Doctor of Philosophy, The University of Melbourne, Parkville, Victoria, Australia.
19. Struhl K. (1989) Trends in Biological Sciences, 14, 137.

## Claims

1. A method for capturing and detecting target DNA which method comprises subjecting said target DNA to a polymerase chain reaction using a set of oligonucleotide primers selected to be complementary to the strands of said target DNA and wherein one of the primers of said set of oligonucleotides bears a first ligand containing a site for a double-stranded DNA-specific binding protein and the other of the primers bears a second ligand or a label, contacting the amplified double-stranded DNA with a solid substrate having the double-stranded DNA-specific DNA binding protein for said first ligand immobilized thereon and detecting the second ligand or label while the amplified double-stranded DNA is bound to said solid substrate to indicate the presence of the amplified double-stranded DNA.

2. A method for capturing and detecting target DNA in a sample, which method comprises amplifying said target DNA, if present in said sample, by a polymerase chain reaction procedure using a first set of oligonucleotide primers selected to be complementary to the strands of said target DNA, and detecting the amplified tarqet DNA by a method according to claim 1 wherein the set of primers employed in said method according to claim 1 is nested between said first set of oligonucleotide primers.

3. The method according to claim 2, wherein the first and second polymerase chain reactions occur in a single reaction mixture.

4. The method according to any one of the preceding claims wherein the binding of the amplified DNA to the solid substrate occurs simultaneously to the binding to a detection complex.

5. The method according to any one of the preceding claims wherein the double stranded DNA-specific DNA binding protein is of the leucine zipper type.

6. The method according to claim 5 wherein the DNA binding protein is GCN4 and/or its derivatives.

7. The method according to claim 6 wherein the DNA binding protein is GST-GCN4.

8. The method according to any one of claims 1 to 4 wherein the double stranded DNA-specific DNA binding protein is of the helix turn helix type.

9. The method according to claim 8, wherein the DNA binding protein is TyrR.

10. The method according to any one of the preceding claims wherein the bound DNA is detected using a detection reagent comprising avidin-peroxidase.

11. The method according to claim 10, wherein the binding moiety for the detection reagent is biotin.

12. The method according to any one of the preceding claims wherein the target DNA is HIV DNA.

13. The method according to any one of the preceding claims wherein the solid substrate coated with the double-stranded DNA-specific DNA binding protein is a well of a microtiter dish.

14. The method according to any one of the preceding claims wherein the solid substrate coated with the double-stranded DNA-specific DNA-binding protein is a single or multiple bead or pin apparatus capable of transferring the amplified product with detection complex to a detection substrate.

15. The method according to any one of the preceding claims wherein the target DNA is cDNA copied by reverse transcriptase from RNA.

16. A test kit for capturing and detecting target DNA in a sample by the method according to any one of the preceding claims, said kit comprising in compartmental form, a first container containing a set of oligonucleotide primers selected to be complementary to the strands of said target DNA and wherein one of the primers of said set of oligonucleotides bears a first ligand containing a site for a double-stranded DNA-specific binding protein and the other of the said primers bears a second ligand or a label; and, in said first container or in a second container, reagents for a polymerase chain reaction, a solid substrate coated with the double-stranded DNA-specific binding protein, and a means for detecting amplified double-stranded DNA bound to said solid substrate.

17. A test kit according to claim 16 further comprising a first set of primers capable of amplifying said target DNA, wherein during polymerase chain reaction said set of primers as defined in claim 16 is nested between said first set of primers.

18. A conjugate for use in a method according to any one of claims 1 to 7 consisting essentially of a support, GST-GCN4 immobilized on said support, and an amplified double-stranded DNA bound at a first end to said GST-GCN4.

19. The conjugate according to claim 18, wherein at a second end the DNA is conjugated to a label.

20. The conjugate according to claim 19, wherein the label is an enzyme.

21. The conjugate according to claim 20 wherein the label is conjugated to the amplified DNA through an avidin-biotin bridge.

## Patentansprüche

1. Verfahren zum Einfangen und Nachweisen von Ziel-DNA, wobei das Verfahren umfaßt: Unterziehen der Ziel-DNA einer Polymerasekettenreaktion unter Verwendung eines Sets von Oligonukleotidprimern, die so ausgewählt sind, daß sie zu den Strängen der Ziel-DNA komplementär sind, und worin einer der Primer des Sets von Oligonukleotiden einen ersten Liganden trägt, der eine Stelle für ein auf doppelsträngige DNA spezifisches Bindungsprotein enthält, und der andere Primer einen zweiten Liganden oder einen Marker trägt, in Kontakt bringen der amplifizierten doppelsträngigen DNA mit einem festen Substrat, das das für doppelsträngige DNA spezifische DNA-Bindungsprotein für den ersten Liganden darauf immobilisiert aufweist, und Nachweisen des zweiten Liganden oder Markers, während die amplifizierte doppelsträngige DNA an das feste Substrat gebunden ist, um das Vorhandensein der amplifizierten doppelsträngigen DNA anzuzeigen.

2. Verfahren zum Einfangen und Nachweisen von Ziel-DNA in einer Probe, wobei das Verfahren umfaßt: Amplifizieren der Ziel-DNA, wenn sie in der Probe vorhanden ist, durch ein Polymerasekettenreaktionsverfahren unter Verwendung eines ersten Sets von Oligonukleotidprimern, die so ausgewählt sind, daß sie zu den Strängen der Ziel-DNA komplementär sind, und Nachweisen der amplifizierten Ziel-DNA durch ein Verfahren nach Anspruch 1, worin das in dem Verfahren nach Anspruch 1 verwendete Set von Primern zwischen das erste Set von Oligonukleotidprimern eingenistet wird.

3. Verfahren nach Anspruch 2, worin die erste und zweite Polymerasekettenreaktion in einer einzigen Reaktionsmischung ablaufen.

4. Verfahren nach einem der vorhergehenden Ansprüche, worin die Bindung der amplifizierten DNA an das feste Substrat gleichzeitig mit der Bindung an einen Nachweiskomplex erfolgt.

5. Verfahren nach einem der vorhergehenden Ansprüche, worin das doppelsträngige DNA-spezifische DNA-Bindungsprotein vom Leucin-Reißverschluß-Typ ist.

6. Verfahren nach Anspruch 5, worin das DNA-Bindungsprotein GCN4 und/oder eines seiner Derivate ist.

7. Verfahren nach Anspruch 6, worin das DNA-Bindungsprotein GST-GCN4 ist.

8. Verfahren nach einem der Ansprüche 1 bis 4, worin das auf doppelsträngige DNA spezifische DNA-Bindungsprotein vom Typ Helix-Biegung-Helix ist.

9. Verfahren nach Anspruch 8, worin das DNA-Bindungsprotein TyrR ist.

10. Verfahren nach einem der vorhergehenden Ansprüche, worin die gebundene DNA unter Verwendung eines Nachweisreagens nachgewiesen wird, das Avidinperoxidase umfaßt.

11. Verfahren nach Anspruch 10, worin die Bindungsgruppe für das Nachweisreagens Biotin ist.

12. Verfahren nach einem der vorhergehenden Ansprüche, worin die Ziel-DNA HIV-DNA ist.

13. Verfahren nach einem der vorhergehenden Ansprüche, worin das mit dem auf doppelsträngige DNA spezifischen DNA-Bindungsprotein beschichtete feste Substrat eine Vertiefung einer Mikrotiterplatte ist.

14. Verfahren nach einem der vorhergehenden Ansprüche, worin das mit dem auf doppelsträngige DNA spezifischen DNA-Bindungsprotein beschichtete feste Substrat eine Vorrichtung mit einer einzelnen oder mehreren Kugeln oder Stiften ist, der in der Lage ist, das amplifizierte Produkt mit Nachweiskomplex in ein Nachweissubstrat zu überführen.

15. Verfahren nach einem der vorhergehenden Ansprüche, worin die Ziel-DNA cDNA ist, die durch reverse Transkriptase aus RNA kopiert ist.

16. Testkit zum Einfangen und Nachweisen von Ziel-DNA in einer Probe durch das Verfahren nach einem der vorhergehenden Ansprüche, wobei der Kit in Form von Teilen umfaßt: einen ersten Behälter mit einem Set von Oligonukleotidprimern, die so ausgewählt sind, daß sie zu den Strängen der Ziel-DNA komplementär sind, und worin einer der Primer des Sets von Oligonukleotiden einen ersten Liganden trägt, der eine Stelle für ein auf doppelsträngige DNA spezifisches Bindungsprotein enthält, und der andere Primer trägt einen zweiten Liganden oder einen Marker; und im ersten Behälter oder in einem zweiten Behälter Reagenzien für eine Polymerasekettenreaktion, ein festes Substrat, das mit dem auf doppelsträngige DNA spezifischen Bindungsprotein beschichtet ist, und ein Mittel zum Nachweisen der amplifizierten doppelsträngigen DNA, die an das feste Substrat gebunden ist.

17. Testkit nach Anspruch 16 ferner umfassend ein erstes Set von Primern, die in der Lage sind, die Ziel-DNA zu amplifizieren, worin während der Polymerasekettenreaktion das Set von Primern wie sie in Anspruch 16 definiert sind, zwischen das erste Set von Primern eingenistet wird.

18. Konjugat zur Verwendung in einem Verfahren nach einem der Ansprüche 1 bis 7 bestehend im wesentlichen aus einem Träger, GST-GCN4 auf dem Träger immobilisiert und einer amplifizierten doppelsträngigen DNA, die an einem ersten Ende an GST-GCN4 gebunden ist.

19. Konjugat nach Anspruch 18, worin an einem zweiten Ende die DNA mit einem Marker konjugiert ist.

20. Konjugat nach Anspruch 19, worin der Marker ein Enzym ist.

21. Konjugat nach Anspruch 20, worin der Marker mit der amplifizierten DNA durch eine Avidin-Biotinbrücke konjugiert ist.

## Revendications

1. Procédé pour la capture et la détection d'ADN cible, lequel procédé comprend la soumission dudit ADN cible à une réaction d'amplification en chaîne par polymérase avec utilisation d'une série d'amorces oligonucléotidiques choisies pour être complémentaires des brins dudit ADN cible et dans lequel l'une des amorces de ladite série d'oligonucléotides porte un premier ligand comportant un site pour une protéine de liaison spécifique d'ADN double brin et l'autre des amorces porte un second ligand ou un marqueur, la mise en contact de l'ADN double brin amplifié avec un support solide sur lequel est immobilisée la protéine de liaison à l'ADN spécifique d'ADN double brin, pour ledit premier ligand, et la détection du second ligand ou marqueur pendant que l'ADN double brin amplifié est fixé audit support solide, pour indiquer la présence de l'ADN double brin amplifié.

2. Procédé pour la capture et la détection d'ADN cible dans un échantillon, lequel procédé comprend l'amplification dudit ADN cible, s'il est présent dans ledit échantillon, par une technique de réaction d'amplification en chaîne par polymérase, avec utilisation d'une première série d'amorces oligonucléotidiques choisies pour être complémentaires des brins dudit ADN cible, et la détection de l'ADN cible amplifié, par un procédé selon la revendication 1, dans lequel la série d'amorces utilisée dans ledit procédé selon la revendication 1 est emboîtée entre ladite première série d'amorces oligonucléotidiques.

3. Procédé selon la revendication 2, dans lequel la première réaction d'amplification en chaîne-par polymérase et la seconde ont lieu dans un seul mélange réactionnel.

4. Procédé selon l'une quelconque des revendications précédentes, dans lequel la fixation de l'ADN amplifié au support solide s'effectue en même temps que la liaison à un complexe de détection.

5. Procédé selon l'une quelconque des revendications précédentes, dans lequel la protéine de liaison à l'ADN, spécifique d'ADN double brin, est du type fermeture à glissière à leucine.

6. Procédé selon la revendication 5, dans lequel la protéine de liaison à l'ADN est GNC4 et/ou ses dérivés.

7. Procédé selon la revendication 6, dans lequel la protéine de liaison à l'ADN est GST-GCN4.

8. Procédé selon l'une quelconque des revendications 1 à 4, dans lequel la protéine de liaison à l'ADN, spécifique d'ADN double brin, est du type hélice-coude-hélice.

9. Procédé selon la revendication 8, dans lequel la protéine de liaison à l'ADN est TyrR.

10. Procédé selon l'une quelconque des revendications précédentes, dans lequel l'ADN fixé est détecté au moyen d'un réactif de détection comprenant l'avidine-peroxydase.

11. Procédé selon la revendication 10, dans lequel le fragment de liaison pour le réactif de détection est la biotine.

12. Procédé selon l'une quelconque des revendications précédentes, dans lequel l'ADN cible est de l'ADN de VIH.

13. Procédé selon l'une quelconque des revendications précédentes, dans lequel le support solide tapissé avec la protéine de liaison à l'ADN, spécifique d'ADN double brin, est un puits d'une plaque de microtitrage.

14. Procédé selon l'une quelconque des revendications précédentes, dans lequel le support solide tapissé avec la protéine de liaison à l'ADN, spécifique d'ADN double brin, est un appareil à simples ou multiples billes ou pointes capable de transférer le produit amplifié avec le complexe de détection sur un substrat de détection.

15. Procédé selon l'une quelconque des revendications précédentes, dans lequel l'ADN cible est de l'ADNc copié par transcriptase inverse à partir d'ARN.

16. Nécessaire d'essai pour la capture et la détection d'ADN cible dans un échantillon par-le procédé selon l'une quelconque des revendications précédentes, ledit nécessaire comprenant, sous forme compartimentée, un premier récipient contenant une série d'amorces oligonucléotidiques choisies pour être complémentaires des brins dudit ADN cible et dans lequel l'une des amorces de ladite série d'oligonucléotides porte un premier ligand comportant un site pour une protéine de liaison spécifique d'ADN double brin et l'autre desdites amorces porte un second ligand ou un marqueur; et, dans ledit premier récipient ou dans un second récipient, des réactifs pour une réaction d'amplification en chaîne par polymérase, un support solide tapissé avec la protéine de liaison spécifique d'ADN double brin, et un moyen pour la détection d'ADN double brin amplifié, fixé audit support solide.

17. Nécessaire d'essai selon la revendication 16, comprenant en outre une première série d'amorces capables d'amplifier ledit ADN cible, dans lequel, pendant la réaction d'amplification en chaîne par polymérase, ladite série d'amorces, telle que définie dans la revendication 16, est emboîtée entre ladite première série d'amorces.

18. Conjugué pour utilisation dans un procédé selon l'une quelconque des revendications 1 à 7, consistant essentiellement en un support, de la GST-GCN4 immobilisée sur ledit support, et un ADN double brin amplifié, fixé, à une première extrémité, à ladite GST-GCN4.

19. Conjugué selon la revendication 18, dans lequel, à une seconde extrémité, l'ADN est conjugué avec un marqueur.

20. Conjugué selon la revendication 19, dans lequel le marqueur est une enzyme.

21. Conjugué selon la revendication 20, dans lequel le marqueur est conjugué avec l'ADN à amplifier par un pont avidine-biotine.
